# EUROPEAN PATENT SPECIFICATION

(11) **EP 3 062 764 B1**
(45) Date of publication and mention of the grant of the patent: **08.12.2021**
(21) Application number: 14796540.4
(22) Date of filing: 29.10.2014
(51) Int. Cl.: A61K 8/06, A61K 8/34, A61K 8/39, A61Q 17/00, A61Q 19/00

(54) **STABILISED MULTIPLE EMULSIONS AS SKIN PROTECTION PRODUCT**
STABILISIERTE MULTIPLE EMULSIONEN ALS HAUTSCHUTZPRODUKT
EMULSIONS MULTIPLES STAILISÉES EN TANT QUE PRODUITS DESTINÉS À PROTÉGER LA PEAU

(30) Priority: 31.10.2013 DE 102013222164
(43) Date of publication of application: 07.09.2016
(73) Proprietor: Deb IP Limited, Denby, Derbyshire DE5 8JZ (GB)
(72) Inventor: HEISLER, Eckhard, 47608 Geldern (DE); MANGEN, Thomas, 40545 Dusseldorf (DE); KAMPS, Nicole, 47807 Krefeld (DE)
(74) Representative: Elsy, David
(86) International application number: PCT/GB2014/053205
(87) International publication number: WO 2015/063471

(56) References cited:
- EP-A2- 2 253 305
- WO-A1-91/12880
- WO-A1-96/13249
- WO-A1-96/13249
- DE-A1-102008 061 044

## Description

The invention relates to stabilised multiple emulsions, methods for the stabilisation of multiple emulsions and the non-therapeutic use of multiple emulsions as skin protection products, in particular for protection against aqueous noxa.

Skin protection products are protection agents against skin damage, which can for example be caused through climatic influences, water and aqueous solutions, chemicals, and especially industrial contamination such as hazardous or very dirty working materials. Such skin protection products, which are in particular intended to prevent or at least reduce the effect of working materials on the skin, coat the skin with a so-called barrier film, which then forms an additional artificial protective barrier against irritating and noxious substances.

Components that contribute towards forming an artificial barrier film in a cosmetic product often consist of paraffin hydrocarbons such as mineral oils, Vaseline etc., but also mineral and vegetable wax including silicone oils and silicone wax, and not least natural or synthetic polymers such as for example alginates, which can be used in cosmetic formulations for this purpose. Skin protection compounds are offered on the market in the most varied preparation forms, whereby skin ointments, skin creams, skin lotions, skin oils and skin gels represent the most important. Skin creams and skin lotions are primarily based on emulsions of the O/W (oil in water) or W/O type (water in oil). The main components of the oil phase (also fat or lipid phase) can then be fat alcohols, fatty acids, fatty acid esters, wax, Vaseline and paraffin, but also other fats and oils of mostly natural origins. The aqueous phase can, amongst others, contain water soluble care ingredients that are moisture regulating or moisture retaining.

With emulsions of the type O/W the fat phase is enclosed by the water phase and the character of the O/W emulsion is primarily inferred by the water phase, so that direct influence can be applied onto the hydrolipid layer of the skin. The use of emulsions of the type W/O however creates a protective layer on the skin, which is inferred through the fat phase of the emulsion. It further supports the collection of moisture on the skin.

Apart from the emulsions of an O/W or W/O type commonly used for skin protection products, multiple emulsions are also described for the production of cosmetic and pharmaceutical products. Such multiple emulsions consist of emulsions of emulsions, the most important representatives of which are multiple water/oil/water (W/O/W) and oil/water/oil (O/W/O) emulsions, often described in patent literature.

DE 41 31 678 A1 for example describes multiple emulsions that can be used in cosmetic skin care products, but also in medical topical preparations. Water and aqueous solutions of moderately skin irritating substances can cause cumulatively toxic contact eczema (attrition dermatitis) after repeated skin contact over an extended period of time. To protect the skin against such exposure water-insoluble barrier preparations are normally used, which form a protective layer on the skin. A disadvantage of such preparations is, however, that the natural water vapour emission via the skin is negatively affected. Due to the heat and moisture block connected with the same the acceptance of the use of such products by employees who come into repeated daily contact with hazardous aqueous skin substances is reduced. Improved acceptance exists for hydrophilic formulations of the type O/W (oil in water), which derive their protective effect from a high content of silicone compounds.

Silicones, such as silicone oils and silicone wax, are excellent barrier agents that have particular stability in skin protection agents. They are heat resistant and extremely resistant towards the effects of extreme environmental influences such as for example caustic chemicals. In addition such silicone preparations are very hydrophobic and harmless for the skin, as they are physiologically compatible, i.e. not harmful to health, and even skin compatible. Thanks to the low surface tension of silicone oils they are easily distributed on the skin. It is also of advantage that silicone layers represent no risk of heat block on the skin, unlike paraffin, Vaseline etc.

DE 4131678 A1 also describes a skin protection emulsion, which contains cyclomethicone as the silicone compound, whereby the W/O/W emulsion is created by using ethoxylated fat alcohols as emulsifiers.

One disadvantage of such silicone containing preparations is however that these preparations can leave residues on objects such as for example material or work pieces when such work pieces are transferred to a subsequent working step by hand. Employees can for example not use such silicone containing skin protection agents when handling work pieces that are to be sprayed, as these silicone residues are very difficult to remove and will have a strongly detrimental effect on the further processing of these work pieces, such as for example during spraying or vulcanising. This means that silicone containing skin protection products cannot be used, in particular within the automotive, spraying and rubber processing industries, despite their excellent protective effect and acceptance.

EP 1427381 A1 discloses skin protection agents produced with the aid of W/OW emulsions, which are formed by means of an emulsifier mixture consisting of polyolpoly-12-hydroxy stearates in combination with an alkyl and/or alkylene glucoside and a fat alcohol and/or partial glycerides, and possibly additional co-emulsifiers, in particular at least one ethoxylated dipolyhydroxy stearate as the co-emulsifier. These skin protection agents are very effective in protecting against water and aqueous, skin irritating solutions.

One advantage of multiple emulsions, in particular of W/O/W emulsions, is their particular sensitivity. W/O/W emulsions are particularly easy to apply, which means that the oil component can be distributed on and into the skin particularly well without however leaving a greasy feeling on the skin. In addition the outer water phase ensures a direct, if short-term moistening of the skin, whilst the water component, which is included within the oil phase, supports the long-term supply of moisture to the skin. A further advantage is the possibility of transporting active substances to specific skin layers in a more selective way. The use of multiple emulsions, in particular of W/O/W emulsion, in skin protection products poses particular challenges to the expert. Contrary to the typically used O/W (oil in water) or W/O (water in oil) emulsions, where only the stability of the simple emulsion needs to be guaranteed, the expert is faced with the challenge of maintaining this multiplicity of the emulsion in a stable condition for multiple emulsions over lengthy storage periods of months, if not years. Multiple emulsions react very sensitively to changes in their composition. A W/O/W emulsion will for example quickly turn into a W/O emulsion following an incorrect selection of components. In addition the stability of the composition must be guaranteed. Phase separation and discolouration are not acceptable for high-value skin protection products. However, the expert is clearly restricted in his choice of components, as it must be guaranteed at all times that the substances used will be accepted by the user of the skin protection products.

Due to the high craft skill requirements posed to the expert by multiple emulsions there continues to be a requirement for new multiple emulsions comprising high stability, which are suitable for use as skin protection products.

It was therefore the purpose of this invention to provide multiple emulsions, in particular W/O/W emulsions, with high stability and particularly good multiplicity, in particular over long storage periods, which also have good effectiveness with regard to protection against water and aqueous, skin irritating solutions. The purpose was further to provide multiple emulsions with a high cosmetic acceptance, such as for example skin care products.

The objects of the invention will be described hereafter by way of examples without limiting the invention to the embodiment examples. Wherever ranges, general formulas or compound classes are mentioned hereafter, these should be understood to include not only the relevant ranges or groups explicitly mentioned, but also all part ranges and part groups of compounds that can be obtained by removing individual values (ranges) or compounds. Wherever documents are cited as part of this description their content, in particular with regard to the facts in connection with which the document was cited, shall be incorporated into the disclosure content of this invention in its entirety. Unless stated differently all percentage information consists of weight percentages. Unless stated differently all pH values were determined with the usual commercial pH meters based on potentiometry. Wherever average values are stated hereafter these consist, unless stated differently, of weight averages. Wherever parameters recorded through measurements are stated hereafter these measurements, unless stated differently, were taken at a temperature of 25°C and a pressure of 101.325 Pa.

Surprisingly it has been found that those skin protection agents form particularly stable multiple emulsions, that
a) comprise 0.2-10% w/w of at least one aliphatic, unbranched diol with 2 to 4 carbon atoms in the hydrocarbon chain,
b) 0.1-1.9w/w of at least one further branched or unbranched aliphatic diol with 4 to 12 carbon atoms in the hydrocarbon chain that is different from a), whereby the hydrocarbon chain may be interrupted by an oxygen atom,
c) 0.5-10% w/w of at least one single-value aliphatic alcohol with 12 to 30 carbon atoms in the hydrocarbon chain,
   whereby excellent multiplicity as well as particularly good stability of the W/O/W emulsion is given with such a combination. Preferred skin protection agents also contain, in addition to components a), b) and c),
d) at least one a further component that carries at least one OH function, as such emulsions have particularly good multiplicity and stability.

The diols listed under a) include all saturated, unbranched hydrocarbons with 2 to 4 carbon atoms in the hydrocarbon chain that carry two functional OH groups. The diols listed under a) preferably comprise no further functional groups. Particularly preferably these are 1,2-propandiol, 1,3-propandiol, 1,2-ethandiol, 1,2-butandiol, 1,3-butandiol and/or 1,4-butandiol, particularly preferably 1,2-propandiol, as the latter has been found to be of particular advantage.

The diols listed under b) include all branched or unbranched aliphatic diols with 4 to 12, preferably 6 to 10 carbon atoms in the hydrocarbon chain of a), that are different from those listed under a), whereby the hydrocarbon chain may be interrupted by an oxygen atom. The diols of the component b) are particularly preferably selected from 1,2-alkan diols with 6 to 12 carbon atoms and/or 1,3-alkan diols with 4 to 12 carbon atoms and/or α-ω-alkan diols with 6 to 12 carbon atoms, also the two alcohol functions on the relevant first and last carbon atom of the longest hydrocarbon chain, and/or monoalkyl glycerines with 4 to 12 carbon atoms, and most particularly preferably the diols of the component b) are selected from 1,2-hexandiol, 1,3-hexandiol, 1,6-hexandiol, 1,2-octandiol (caprylyl glycol), 1,3-octandiol, 1,8-octandiol, 1,2-decandiol, 1,10-decandiol, 1,2-dodecandiol, glyceryl caprylate, methyl propane diol, ethyl hexyl glycerine, hexylene glycol, in particular 1,2-hexandiol, 1,6-hexandiol, 1,2-octandiol (caprylyl glycol), 1,8-octandiol, methyl propane diol, ethyl hexyl glycerine and/or hexylene glycol. In a particularly preferred embodiment a mixture of 1,2-hexandiol and 1,2-octandiol is used as component b), preferably in quantities of 0.1 to 1.9% w/w.

The alcohols listed under c) include all saturated or unsaturated aliphatic alcohols with 12 to 30, preferably 16 to 18 carbon atoms in the hydrocarbon chain that carry a functional OH group. Preferred are the alcohols of component c), namely linear, saturated, preferably primary alkanols. Preferred alcohols of component c) comprise no further functional groups. Particularly preferably these are lauryl alcohol (dodecan-1-ol), hexadecan-1-ol (cetyl alcohol), octadecan-1-ol (stearyl alcohol), hexacosan-1-ol (ceryl alcohol), triacontan-1-ol (myricyl alcohol), particularly preferably a mixture of cetyl alcohol and stearyl alcohol (cetearyl alcohol), as the latter has been found to be of particular advantage.

The further components listed under d) include all components with at least one, preferably precisely one, OH function, preferably with 3 to 30 carbon atoms, that may possibly carry further functional groups. The components listed under d) preferably consist of compounds comprising of at least one unsaturated hydrocarbon ring with 6, 7 or 8 carbon atoms. Particularly preferable examples of compounds of the component d) are bisabolol, 1,2-tropolone, capryl hydroxamic acid, glycerine, phenoxy ethanol, alpha-tocopherol, phenethyl alcohol and/or trideceth-8 (ethoxylated isotridecanol, for example obtainable from Sasol). Component d) preferably comprises at least bisabolol, alpha-tocopherol, phenethyl alcohol and/or phenoxy ethanol. Component d) preferably comprises at least bisabolol. Component d) also preferably comprises at least alpha-tocopherol, phenethyl alcohol and/or phenoxy ethanol.

Component a) is present in quantities of 0.2 to 10% w/w, preferably of 0.5 to 5% w/w, and particularly preferably of 1 to 2.5% w/w in relation to the total weight of the skin protection agent. Component b) is present in quantities of 0.1 to 1.9% w/w, preferably of 0.2 to 1.7% w/w, and particularly preferably of 0.3 to 1.5% w/w in relation to the total weight of the skin protection agent. Component c) is present in quantities of 0.5 to 10% w/w, preferably of 1 to 5% w/w, and particularly preferably of 1.5 to 3% w/w in relation to the total weight of the skin protection agent. If further component d) is included in the skin protection agent this is preferably present in quantities of 0.1 to 5% w/w, preferably of 0.2 to 3% w/w, and particularly preferably of 0.3 to 2% w/w in relation to the total weight of the skin protection agent. trials have shown that the characteristics of the skin protection agent, in particular the stability over a period of several weeks, clearly worsened when individual components, in particular component b), are used in quantities outside of the preferred quantity ranges. In a most particularly preferred embodiment a mixture of 1,2-hexandiol and 1,2-octandiol, c) ceteary alcohol, and optionally an alcohol of the component d), preferably at least bisabolol, is used in the skin protection agent that is a multiple emulsion, preferably a W/O/W, a combination of the components a) 1,2-propandiol, b).

A preferred embodiment of the skin protection agent according to the invention further comprises at least one emulsifier. Particularly preferably such skin protection agents comprise at least one emulsifier in a quantity of 1 to 25% w/w, preferably 2 to 15% w/w, more preferably 4 to 12% w/w, particularly preferably 5 to 10% w/w in relation to the total composition. Emulsifiers in the sense of the present invention can in principle be understood as all those components known to the expert that will positively influence the sensitivity of a composition, and thus for example make the epidermis of the skin more flexible and softer. Preferred emulsifiers can be selected from the group PEG-30 glyceryl cocoate, PEG-6 caprylic/capric glyceride, sorbitan sesquicaprylate, sucrose cocoate, isoamyl cocoate polyglyceryl-3 caprate, PPG-3 myristyl ether, PPG-11 stearyl ether, cetearyl isononanoate, cetyl ethyl hexanoates, caprylic/capric triglyceride, decal cocoates, diethylhexyl carbonate, decyl oleate, PPG-15 stearyl ether, octyl dodecanol, isocetyl palmitate, isohexadecane, cetearyl ethyl hexanoate, isopropyl myristate, oleyl erucate, ethyl hexyl palmitate, ethyl hexyl stearate, isopropyl palmitate, PPG-14 butyl ether, triisostearine, C12-15 alkyl benzoate, cetyl ricenoleate, glyceryl ricenoleate, glyceryl stearate, isocetyl palmitate, isocetyl stearate, tocopheryl linoleate, methylheptyl isostearate and mixtures of these, particularly preferred is isoamyl cocoate. These skin protection agents with emulsifiers achieve extraordinary good spreading characteristics of the total composition and particularly excellent absorption characteristics on the skin, whilst displaying high stability of the composition in combination with further components a), b), c) and possibly d).

In a particularly preferred embodiment the W/O/W emulsions according to the invention further include an emulsifier mixture. Particularly preferred is a combination of polyolpoly-12-hydroxy stearates with an alkyl and/or alkylene glucoside and a fat alcohol and/or partial glycerides and possible further co-emulsifiers, in particular preferably at least one ethoxylated dipolyhydroxy stearate. Skin protection agents with such emulsifier systems do not only form stable multiple emulsions, but display excellent care characteristics at the same time.

According to the invention the emulsifier mixture is preferably used in quantities of 1 to 25% w/w, preferably 5 to 15% w/w, in relation to the total composition of the skin protection agent, for a skin protection agent.

The emulsifier mixture preferably comprises at least one polyolpoly-12-hydroxy stearate as emulsifier component, preferably in a quantity of 1 to 5% w/w, in relation to the total composition of the skin protection agent, whereby polyglycerinpoly-12-hydroxy stearates, commercially available under the brand name of Dehymuls^{®} PGPH distributed by Henkel KGaA, Dusseldorf, are particularly preferred as the emulsifier component.

The addition of further co-emulsifiers to the emulsifier mixture is of advantage. The emulsifier mixture can thus be admixed with at least one further ethoxylated dipolyhydroxy stearate, in particular in a usage quantity of 0.1 to 1.0% w/w, once again in relation to the total composition of the skin protection agent, whereby the co-emulsifiers PEG-30 dipolyhydroxy stearate, commercially available under the name of ARLACEL P135, are particularly preferred. Further co-emulsifiers that can be used are hydrophilic emulsifiers known to the expert, which can also be admixed with the emulsifier mixture at a usage quantity of 0.1 to 1.0% w/w, in relation to the total quantity of the agent. One example to be mentioned here is PEG-40 stearate.

Very good effectiveness compared with aqueous noxa and very good cosmetic acceptance (comparable to a light skin care cream) can in particular be present when the content of unpolar oils does not exceed 20% w/w, preferably 15% w/w, and particularly preferably 10% w/w, in relation to the total weight of the skin protection agent.

Unpolar oils to be used are preferably the usual oils used for cosmetic or pharmaceutical preparations that coat the skin with a protective or barrier film, in particular paraffin hydrocarbons such as mineral oils, for example Vaseline etc., including mineral and vegetable wax, where these will not result in undesirable residues on materials and work pieces, as is for example the case with the silicone containing preparations. Thanks to the polarity of these oils an excellent protective effect with regard to harmful hydrophilic skin substances can be achieved. In addition these unpolar oils can also comprise additives such as for example isopropyl palmitate or isopropyl myristate or other additives known to the expert to increase spreadability and cohesiveness.

It has further been found that an addition of preferably 0.1 to 5 % w/w, preferably 0.5 to 2% w/w, and particularly preferably 0.5 to 1.5% w/w, of bisabolol as component d) realises an improvement of the barrier, in particular also for skin that is already damaged, thus realising an effective support of skin regeneration. Of further advantage is the addition of natural vegetable tannins, preferably in a quantity of 0.1 to 5% w/w, in relation to the total quantity of the skin protection agent, whereby hamamelis virginiana is particularly preferred as a tannin.

The positive effects of skin protection, skin regeneration and of the cosmetic acceptance of formulations with a combination of emulsifier mixture, unpolar oils, and in particular of bisabolol, are already known to the expert. It thus comes as a particular surprise that W/O/W emulsions comprising a combination of components a), b) and c) stabilise the emulsions particularly strongly, but without reducing the positive effects in the sense of skin protection, skin regeneration and of cosmetic acceptance. This is especially surprising in particular because alcohol components in cosmetic and skin care compositions are usually known as preservatives or suchlike. Components a), b) and c) are generally not known for their caring effect, nor would the expert expect that such a combination of components a), b) and c) would result in a stabilisation of multiple emulsions.

Preferred skin protection agents according to the invention also contain water, preferably in quantities of 30 to 90% w/w, more preferably of 50 to 85% w/w, more preferably of 60 to 80% w/w, and particularly preferably of 68 to 78% w/w in relation to the total weight of the skin protection agent. Such W/O/Ws according to the invention, especially those with a water content of more than 60% w/w, display particularly good multiplicity and are particularly stable.

Very particularly preferable skin protection agents according to the invention are therefore W/O/W emulsions comprising
a) at least one aliphatic unbranched diol with 2 to 4 carbon atoms in the hydrocarbon chain, preferably selected from 1,2-propandiol, 1,3-propandiol, 1,2-ethandiol, 1,2-butandiol, 1,3-butandiol and/or 1,4-butandiol, particularly preferably 1,2-propandiol, preferably in quantities of 0.2 to 10% w/w, preferably of 0.5 to 5% w/w, and particularly preferably of 1 to 2.5% w/w,
b) at least one further aliphatic diol with 4 to 12 carbon atoms that is different from the hydrocarbon chain of a), whereby the hydrocarbon chain may be interrupted by an oxygen atom, preferably selected from 1,2-alkan diols with 6 to 12 carbon atoms and/or 1,3-alkan diols with 4 to 12 carbon atoms and/or α-ω-alkan diols with 6 to 12 carbon atoms and/or monoalkyl glycerines with 4 to 12 carbon atoms, particularly preferably selected from 1,2-hexandiol, 1,3-hexandiol, 1,6-hexandiol, 1,2-octandiol (caprylyl glycol), 1,3-octandiol, 1,8-octandiol, 1,2-decandiol, 1,10-decandiol, 1,2-dodecandiol, glyceryl caprylate, methyl propane diol, ethyl hexyl glycerine, hexylene glycol, in particular from 1,2-hexandiol, 1,6-hexandiol, 1,2-octandiol (caprylyl glycol), 1,8-octandiol, methyl propane diol, ethyl hexyl glycerine and/or hexylene glycol, preferably b) in quantities of 0.1 to 1.9% w/w, preferably of 0.2 to 1.7% w/w, and particularly preferably of 0.3 to 1.5% w/w,
c) at least one single-value aliphatic alcohol with 12 to 30, preferably 16 to 18 carbon atoms in the hydrocarbon chain, preferably linear, saturated, preferably primary alkanols without further functional groups, particularly preferably selected from lauryl alcohol, cetyl alcohol, stearyl alcohol, ceryl alcohol and/or myricyl alcohol, particularly preferably from cetyl alcohol and stearyl alcohol, preferably in quantities of 0.5 to 10% w/w, preferably of 1 to 5% w/w and particularly preferably of 1.5 to 3% w/w,
d) optionally at least one further component with at least one, preferably precisely one, OH function, preferably with 3 to 30 carbon atoms, which may carry further functional groups, preferably consisting of compounds with at least one unsaturated hydrocarbon ring with 6, 7 or 8 carbon atoms, particularly preferably compounds of the component d), selected from bisabolol, 1,2-tropolone, capryl hydroxamic acid, glycerine, phenoxy ethanol, alpha-tocopherol, phenethyl alcohol and/or trideceth-8, preferably at least bisabolol, alpha-tocopherol, phenethyl alcohol and/or phenoxy ethanol, preferably in quantities of 0.1 to 5% w/w, preferably of 0.2 to 3% w/w, and particularly preferably of 0.3 to 2% w/w,
e) an emulsifier mixture, preferably selected from PEG-30 glyceryl cocoate, PEG-6 caprylic/capric glyceride, sorbitan sesquicaprylate, sucrose cocoate, isoamyl cocoate polyglyceryl-3 caprate, PPG-3 myristyl ether, PPG-11 stearyl ether, cetearyl isononanoate, cetyl ethyl hexanoates, caprylic/caprice triglyceride, decal cocoates, diethylhexyl carbonate, decyl oleate, PPG-15 stearyl ether, octyl dodecanol, isocetyl palmitate, isohexadecane, cetearyl ethyl hexanoate, isopropyl myristate, oleyl erucate, ethyl hexyl palmitate, ethyl hexyl stearate, isopropyl palmitate, PPG-14 butyl ether, triisostearine, C12-15 alkyl benzoate, cetyl ricenoleate, glyceryl ricenoleate, glyceryl stearate, isocetyl palmitate, isocetyl stearate, tocopheryl linoleate, methylheptyl isostearate and mixtures of the same, particularly preferable is isoamyl cocoate, most particularly preferable is a combination of polyolpoly-12-hydroxy stearates with an alkyl and/or alkylene glucoside and a fat alcohol and/or partial glycerides and possible further co-emulsifiers, particularly preferably at least one ethoxylated dipolyhydroxy stearate, preferably in a quantity of 1 to 25% w/w, preferably 2 to 15% w/w, more preferably 4 to 12% w/w,
f) water, preferably in quantities of 30 to 90% w/w, more preferably of 50 to 85% w/w, more preferably of 60 to 80% w/w, and particularly preferably of 68 to 78% w/w,
g) further components.

With the above listings each combination of characteristics and preferred characteristics should preferably be considered as having been disclosed with any other of the above.

Most particularly preferred skin protection agents according to the invention are also W/O/W emulsions comprising
a) 0.2 to 10% w/w, preferably 0.5 to 5% w/w, and particularly preferably 1 to 2.5% w/w of at least one aliphatic unbranched diol with 2 to 4 carbon atoms in the hydrocarbon chain,
b) 0.1 to 1.9% w/w, preferably 0.2 to 1.7% w/w, and particularly preferably 0.3 to 1.5% w/w of at least one further aliphatic diol with 4 to 12 carbon atoms in the hydrocarbon chain that is different from a),
c) 0.5 to 10% w/w, preferably 1 to 5% w/w, and particularly preferably 1.5 to 3% w/w of at least one single-value aliphatic alcohol with 12 to 30 carbon atoms in the hydrocarbon chain,
d) 0.1 to 5% w/w, preferably 0.2 to 3% w/w, and particularly preferably 0.3 to 2% w/w of at least one further compound with 3 to 30 carbon atoms that carry at least one OH function,
e) 1 to 25% w/w, preferably 2 to 15% w/w, more preferably 4 to 12% w/w of an emulsifier mixture, preferably selected from PEG-30 glyceryl cocoate, PEG-6 caprylic/capric glyceride, sorbitan sesquicaprylate, sucrose cocoate, isoamyl cocoate polyglyceryl-3 caprate, PPG-3 myristyl ether, PPG-11 stearyl ether, cetearyl isononanoate, cetyl ethyl hexanoates, caprylic/capric triglyceride, decal cocoates, diethylhexyl carbonate, decyl oleate, PPG-15 stearyl ether, octyl dodecanol, isocetyl palmitate, isohexadecane, cetearyl ethyl hexanoate, isopropyl myristate, oleyl erucate, ethyl hexyl palmitate, ethyl hexyl stearate, isopropyl palmitate, PPG-14 butyl ether, triisostearine, C12-15 alkyl benzoate, cetyl ricenoleate, glyceryl ricenoleate, glyceryl stearate, isocetyl palmitate, isocetyl stearate, tocopheryl linoleate, methylheptyl isostearate and mixtures of the same, particularly preferable is isoamyl cocoate,
f) 30 to 90% w/w, preferably 50 to 85% w/w, more preferably 60 to 80% w/w, and particularly preferably 68 to 78% w/w water,
g) further components of a quantity of 100% w/w - (sum of quantities of the components a) to f)),
whereby the % w/w each relate to the total weight of the skin protection agent and add up to the sum of all component weights (% w/w), i.e. 100% w/w.

Very particularly preferably the skin protection agents according to the invention are also W/O/W emulsions comprising
a) 0.2 to 10% w/w, preferably 0.5 to 5% w/w, and particularly preferably 1 to 2.5% w/w of at least one diol selected from 1,2-propandiol, 1,3-propandiol, 1,2-ethandiol, 1,2-butandiol, 1,3-butandiol and/or 1,4-butandiol,
b) 0.1 to 1.9% w/w, preferably 0.2 to 1.7% w/w, and particularly preferably 0.3 to 1.5% w/w of at least one further aliphatic diol selected from 1,2-hexandiol, 1,3-hexandiol, 1,6-hexandiol, 1,2-octandiol, 1,3-octandiol, 1,8-octandiol, 1,2-decandiol, 1,10-decandiol, 1,2-dodecandiol, glyceryl caprylate, methyl propane diol, ethyl hexyl glycerine, hexylene glycol, particularly from 1,2-hexandiol, 1,6-hexandiol, 1,2-octandiol (caprylyl glycol), 1,8-octandiol, methyl propane diol, ethyl hexyl glycerine and/or hexylene glycol that is different from a),
c) 0.5 to 10% w/w, preferably 1 to 5% w/w, and particularly preferably 1.5 to 3% w/w of at least one alcohol selected from lauryl alcohol, cetyl alcohol, stearyl alcohol, ceryl alcohol and/or myricyl alcohol,
d) 0.1 to 5% w/w, preferably 0.2 to 3% w/w, and particularly preferably 0.3 to 2% w/w of at least one further compound selected from bisabolol, 1,2-tropolone, capryl hydroxamic acid, glycerine, phenoxy ethanol, alpha-tocopherol, phenethyl alcohol and/or trideceth-8, preferably selected from phenoxy ethanol, alpha-tocopherol, 1,2-tropolone, phenethyl alcohol,
e) 1 to 25% w/w, preferably 2 to 15% w/w, more preferably 4 to 12% w/w of an emulsifier mixture consisting of a combination of polyolpoly-12-hydroxy stearates with an alkyl and/or alkylene glucoside and a fat alcohol and/or partial glycerides and possible further co-emulsifiers, particularly preferably at least one ethoxylated dipolyhydroxy stearate,
f) 30 to 90% w/w, preferably 50 to 85% w/w, more preferably 60 to 80% w/w, and particularly preferably 68 to 78% w/w water,
g) further components in a quantity of 100% w/w - (sum of the quantities of components a) to f)),
whereby the % w/w each relate to the total weight of the skin protection agent and add up to the sum of all component weights (% w/w), i.e. 100% w/w.

Very particularly preferable skin protection agents according to the invention are also W/O/W emulsions comprising
a) 0.2 to 10% w/w, preferably 0.5 to 5% w/w, and particularly preferably 1 to 2.5% w/w 1,2-propandiol,
b) 0.1 to 1.9% w/w, preferably 0.2 to 1.7% w/w, and particularly preferably 0.3 to 1.5% w/w of at least one further aliphatic diol selected from 1,2-hexandiol, 1,6-hexandiol, 1,2-octandiol, 1,8-octandiol, methyl propane diol, ethyl hexyl glycerine and/or hexylene glycol that is different from a),
c) 0.5 to 10% w/w, preferably 1 to 5% w/w, and particularly preferably 1.5 to 3% w/w of at least one alcohol selected from lauryl alcohol, cetyl alcohol and/or stearyl alcohol,
d) 0.1 to 5% w/w, preferably 0.2 to 3% w/w, and particularly preferably 0.3 to 2% w/w of at least one further compound selected from bisabolol, and/or phenoxy ethanol, alpha-tocopherol, 1,2-tropolone and/or phenethyl alcohol,
e) 1 to 25% w/w, preferably 2 to 15% w/w, more preferably 4 to 12% w/w of an emulsifier mixture consisting of a combination of polyolpoly-12-hydroxy stearates with an alkyl and/or alkylene glucoside and a fat alcohol and/or partial glycerides and possible further co-emulsifiers, particularly preferably at least one ethoxylated dipolyhydroxy stearate,
f) 30 to 90% w/w, preferably 50 to 85% w/w, more preferably 60 to 80% w/w, and particularly preferably 68 to 78% w/w water,
g) further components in a quantity of 100% w/w - (sum of the quantities of components a) to f)),
whereby the % w/w each relate to the total weight of the skin protection agent and add up to the sum of all component weights (% w/w), i.e. 100% w/w.

The skin protection agents according to the invention can comprise further optional components normally used in cosmetic and/or dermatologic formulations. Preferred further components can be selected from the group of fragrances and perfumes, preservatives and/or pH adjusting agents such as for example aqueous sodium hydroxyte.

The skin protection agents according to the invention can comprise further components for adjustment purposes, which serve for the treatment, care, cleaning and the protection of the skin, such as for example active cosmetic skin substances (active ingredients). The term active cosmetic skin substances in the sense of this invention includes, for example, ceramides, pseudoceramides, protein hydrolysates of a vegetable or animal origin on a keratin basis, collagen, elastin, wheat, rise, soya, milk, silk, maize, amino acids and amino acid derivatives, inflammation inhibiting active substances, anti-microbial active substances, common antioxidants, vitamins, dexpanthenol, lactic acid, pyrrolidone carboxylic acid, bisabolol and vegetable, yeast and/or algae extracts.

The combination with commonly used organic or inorganic UV filter substances is also possible and is for example considered to be of particular advantage in sun protection preparations, as a dehydration of the skin during use can be effectively prevented and the natural protection function of the skin is retained in this way. In addition further cosmetic excipients and additives that are usual for such preparations can be present. Such excipients are, for example, solubilisers such as ethanol, isopropanol, ethylene glycol, propylene glycol and diethylene glycol. Further components include cosmetic oils of a vegetable and synthetic origin, silicone oils, fats, refating agent, emulsifiers, anionic, zwitterionic, ampholytic and non-ionic surfactants and/or colourings.

Finally the skin protection agents according to the invention can also comprise chelating agent such as EDTA, NTA, β-alanindiacetic acid and phosphonic acid, colourings for dying the cosmetic preparation, opacifiers such as latex, styrol/PVP and styrol-acrylamide copolymers, ceramides, β-glucanes, oligopepides, hyaluronic acid, nacreous pigments such as ethylene englycolmono- and distearates and PEG-3 distearates, pigments, light protection agents, thickening agents or propellants.

In a preferred embodiment skin protection agents according to the invention are free from compounds selected from silicone compounds, dipropyl englycol, parabens and/or alkyl parabens, formaldehyde splitters such as siazolidinyl urea, imidazolidinyl urea and/or DMDM hydantoin, preservatives based on halogen organic compounds such as for example triclosan, and of mixtures of the same.

In a particularly preferred embodiment the skin protection agent is free from silicones and silicone compounds. It is of particular advantage that such skin protection agents have no negative effect on work processes caused by silicone compound residues, for example on materials or work pieces.

In a further particularly preferred embodiment the skin protection agent according to the invention is free from parabens and/or alkyl parabens, particularly preferably free from parabens and alkyl parabens. In accordance with the invention the terms parabens and alkyl parabens particularly also include 4-hydroxy benzoic acid and 4-hydroxy benzoic acid ester such as for example methyl-4-hydroxy benzoate, ethyl-4-hydroxy benzoate, propyl-4-hydroxy benzoate and/or butyl-4-hydroxy benzoate. In an especially preferred embodiment compositions according to the invention can be free from silicone compounds and free from parabens as well as free from alkyl parabens.

In a particularly preferred embodiment the skin protection agent is free from preservatives as long as these do not fall under one of the components a), b) and c) of the invention and serve for the stabilisation of the multiple emulsion. With other words, a preferred skin protection agent according to the invention is free from preservatives, but can still comprise components in the sense of this invention that are known to the expert as components of preservatives, but are used in accordance with the invention as components a), b) and c). The skin protection agent is preferably free from preservatives that exceed an incidental overlapping with one of the components a), b) and c).

The compositions according to the invention preferably have a viscosity of 5,000 to 10,0000 mPas s, particularly preferably of 6,000 to 50,000 mPas s, particularly preferably of 8,000 to 25,000 mPas s measured with a viscosimeter by company Brookfield Engineering Inc., namely a Brookfield RVT rotation viscosimeter with a spindle set 4-6, preferably 5.

### Production methods

The skin protection agents according to the invention can be produced in any preferred way. Compositions according to the invention are preferably produced in accordance with the method of the invention described below.

A further object of the present invention therefore consists of methods for the production of stable W/O/W emulsions, comprising the components of
a) 0.2-10% w/w of at least one aliphatic unbranched diol with 2 to 4 carbon atoms in the hydrocarbon chain,
b) 0.1-1.9w/w of at least one further aliphatic diol with 4 to 12 carbon atoms in the hydrocarbon chain that is different from a), whereby the hydrocarbon chain may be interrupted by an oxygen atom, and
c) 0.5-10% w/w of at least one single-value aliphatic alcohol with 12 to 30 carbon atoms in the hydrocarbon chain,
   whereby excellent multiplicity as well as a particularly good stability of the W/O/W emulsion is given with such methods. Preferred methods for the production of stable W/O/W emulsions, comprise, in addition to components a), b) and c), also
d) at least one further component that carries at least one OH function, as emulsions produced in this way display particularly good multiplicity and stability.

In a particularly preferred embodiment the W/O/W emulsions according to the invention, comprising components a), b), c) and optional d), are created using an emulsifier mixture of polyolpoly-12-hydroxy stearates in combination with an alkyl and/or alkylene glucoside and a fat alcohol and/or partial glycerides and possible further co-emulsifiers, in particular at least one ethoxylated dipolyhydroxy stearate as co-emulsifier.

Skin protection agents produced in accordance with the invention using multiple emulsions in particular provide excellent protection against aqueous noxa, so that good skin protection is guaranteed even for wet work according to TRGS 531. Such skin protection agents can therefore be used to advantage at workplaces with aqueous skin exposure, for example in the food processing, metal processing, rubber processing industries, in hospitals and in the agriculture and forestry industries, but also during corresponding leisure, hobby and household activities such as for example garden and cleaning work.

Each of the skin protection agents according to the invention can be produced with these methods by adjusting the relevant quantities and the use of the corresponding active substances.

Also an object of the present invention is the use of a combination of
a) 0.2-10% w/w of at least one aliphatic unbranched diol with 2 to 4 carbon atoms in the hydrocarbon chain,
b) 0.1-1.9w/w of at least one further branched or unbranched aliphatic diol with 4 to 12 carbor atoms in the hydrocarbon chain that is different from of a), whereby the hydrocarbon chain may be interrupted by an oxygen atom,
c) 0.5-10% w/w of at least one single-value aliphatic alcohol with 12 to 30 carbon atoms in the hydrocarbon chain, and
d) optionally at least one further component that carries at least one OH function, in skin protection agents for the stabilisation of W/O/W emulsions.

One object of the present invention is the non-therapeutic use of a combination of
a) 0.2-10% w/w of at least one aliphatic unbranched diol with 2 to 4 carbon atoms in the hydrocarbon chain,
b) 0.1-1.9w/w of at least one further branched or unbranched aliphatic diol with 4 to 12 carbon atoms in the hydrocarbon chain that is different from of a), whereby the hydrocarbon chain may be interrupted by an oxygen atom,
c) 0.5-10% w/w of at least one single-value aliphatic alcohol with 12 to 30 carbon atoms in the hydrocarbon chain, and
d) optionally at least one further component that carries at least one OH, in skin protection agents for improved protection against aqueous noxa.

Skin protection agents according to the invention, or produced according to the invention, are preferably applied in the form of a cream, lotion or gel onto the human skin. A further object of the present invention is therefore the use of skin protection agents according to the invention for the topical application on the skin. Each skin area, such as for example the face, hands, lower arms and even the entire human body can be treated with the compositions according to the invention. The hands, lower arms and face, in particular the hands and lower arms, are preferably treated with the compositions according to the invention. Every possible form of applying the compositions to the skin is included in the present invention. Examples of possible applications are the application by hand and with accessories and those of moisturising, rubbing in, spraying on, massaging in.

A further object of the present invention is therefore the non-therpeutic use of skin protection agents according to the invention as a skin cream, particularly as a cream for hands and lower arms.

A further object of the present invention is the cosmetic use of the skin protection agents according to the invention for non-therapeutic skin care purposes.

A further object of the present invention is the cosmetic use of the skin protection agents according to the invention as moisturisers for the skin. The cosmetic use can preferably involve compositions according to the invention as long-lasting skin moisturisers. A long-lasting skin moisturiser can be present according to the invention when skin moisture measurements confirm the moisturising effect.

A further object of the present invention is the non-therapeutic, cosmetic use of the skin protection agents according to the invention for the reduction of a rough, dry and/or tight skin feel.

A reduction of a rough, dry and/or tight skin feel can be present according to the invention when the subjective perception of the respective test person confirms this.

Skin is severely affected by environmental and work related influences, in particular during the working day. A further object of the present invention is therefore the non-therapeutic, cosmetic use of skin protection agents according to the invention for stressed skin. A preferred object of the present invention is also the non-therapeutic, cosmetic use of skin protection agents according to the invention for protecting stressed skin.

A further object of the present invention is the non-therapeutic, cosmetic use of the skin protection agents according to the invention for normal skin.

A further object of the present invention is the non-therapeutic, cosmetic use of the skin protection agents according to the invention for daily use.

A further object of the present invention is the non-therapeutic, cosmetic use of the skin protection agents according to the invention for daily use under normal working conditions. Normal working conditions in the sense of this invention should for example be understood as office work, production, sales, trade activities, simple laboratory activities, external working conditions under normal climate conditions such as for example at temperatures between 0°C and 30°C, occasional contact with water or detergents, the occasional wearing of gloves which may result in skin softening, and all everyday working activities where normal influences, as opposed to extreme influences such as for example extreme cold of substantially below 0°C, extreme heat of substantially above 40°C, permanent skin contact with chemicals such as formaldehyde, active chemical substances or detergents, or UV radiation can have an effect on the skin.

Skin protection agents according to the invention provide excellent protection against aqueous noxa, so that good skin protection is also provided for wet work according to TRGS 531. Such skin protection agents can therefore be used to advantage at workplaces involving aqueous skin exposure, for example in the food processing, metal processing and rubber processing industries, in hospitals, care homes, and in the agricultural and forestry industries, but also for corresponding leisure, hobby and household activities, for example garden and cleaning work.

In addition the skin protection agents according to the invention offer excellent protection against aqueous noxa for skin areas that are particularly stressed more than normal due to confinement in bed, one-sided stresses or pressure sores caused in some other way. The skin protection agents are particularly suitable as a bedsore treatment here, in particular as a treatment of stage 1 bedsores. The skin protection agents according to the invention in particular offer excellent protection against napkin dermatitis. The use of the skin protection agents according to the invention are therefore particularly preferable for example on hospital wards, in care homes, nursing wards and/or for private nursing care, for protection against bedsores and against napkin rash.

The said uses can of course also overlap or occur simultaneously. One preferred use can therefore be the, preferably daily, use of the skin protection agents according to the invention as a skin cream for the topical application on the skin, in particular as a cream for the hands and lower arms, for stressed or normal skin and for use during normal working conditions and/or for skin care purposes and/or as a moisturiser, in particular a long-lasting moisturiser, for the skin and/or for the protection of stressed skin and/or for the reduction of a rough, dry and/or tight skin feel.

The uses according to the invention include all preferred embodiments of the skin protection agents of the invention described above.

The invention will now be described with reference to examples. These descriptions represent examples only and do not restrict the present invention in any way.

### Examples:

Various W/O/W emulsions were produced and examined with regard to their multiplicity and their storage stability at different temperatures over a period of 6 weeks.

The multiplicity of drops was examined under the microscope and evaluated in line with the following scale:
- almost none or none of the drops show multiplicity
+ the majority of the drops show multiplicity
++ almost all of the drops show multiplicity
+++ all of the drops show multiplicity

| **Combination of components** | **Composition** | **Cone. components b) in % w/w** | **Multiplicity of the W/O/W** | **RT** | **40°C** | **4°C** |
|---|---|---|---|---|---|---|
| a), b), c) and d) | 1,2-propandiol 1,2-hexandiol, caprylyl glycol, cetearyl alcohol bisabolol tropolone | 1.00% | ++ | stable | stable | stable |
| a), b), c) | 1,2-propandiol caprylyl glycol, cetearyl alcohol glycerine | 1.20% | + | stable | stable | stable |
| a), b), c) | 1,2-butandiol glyceryl caprylate, methyl propane diol cetearyl alcohol | 1.20% | + | stable | stable | stable |
| a), b), c) and d) | 1,3-propandiol caprylyl glycol, hexylene glycol, cetearyl alcohol phenoxy ethanol | 1.00% | ++ | stable | stable | stable |
| a), b), c) | 1,2-propandiol caprylyl glycol, ethyl hexyl glycerine cetearyl alcohol | 1.00% | ++ | stable | stable | stable |
| a), b), c) and d) | 1,2-propandiol caprylyl glycol, ethyl hexyl glycerine, cetearyl alcohol alpha-tocopherol | 2.00% | + | Cracks in the product, first oil formation | 3 phases | grainy, liquid on surface |
| a), b), c) and d) | 1,2-propandiol ethyl hexyl glycerine, cetearyl alcohol tocopherol phenoxy ethanol, | 1.00% | +++ | stable | stable | stable |
| a), b), c) and d) | 1,2-propandiol caprylyl glycol, 1,2-hexanediol, cetearyl alcohol methyl benzyl alcohol | 1.00% | + | stable | stable | stable |
| a), b), c) and d) | 1,2-propandiol ethyl hexyl glycerine, cetearyl alcohol alpha-tocopherol | 1.00% | + | stable | stable | stable |
| a), b), c) and d) | ethyl hexyl glycerine lauryl alcohol, phenethyl alcohol, alpha-tocopherol | 1.10% | + | stable | stable | stable |
| a), b), c) and d) | 1,2-ethandiol Caprylyl glycol, cetearyl alcohol phenoxy ethanol, glycerine | 1.50% | +++ | stable | Slight separation at bottom | Slight separation at top |
| c) (Comparison trial) | cetearyl alcohol | - | - | stable | stable | stable |
| a) and b) (comparison trial) | 1,2-propandiol, caprylyl glycol | 1.00% | - | stable | Separates, liquid at bottom | stable |
| b), c) and d) (comparison trial) | methyl propandiol, glyceryl, caprylate cetearyl alcohol phenethyl alcohol | 1.50% | - | stable | Liquid separation on the surface | Liquid separation on the surface |
| a) and c) (comparison trial) | 1,2-propandiol, cetearyl alcohol | - | - | stable | stable | stable |

The comparison trials clearly show that only those skin protection agents according to the invention that comprise the components a), b) and c) will result in multiple W/O/W emulsions that are also stable. The absence of at least one of the components will result either in emulsions that are not multiple, and thus immediately disintegrate into a W/O or O/W emulsion and/or into skin protection agents that are less stable during storage.

## Claims

1. Skin protection agent, in the form of a water - in oil- in water (W/O/W) emulsion, comprising:
a) 0.2-10% w/w of_at least one aliphatic unbranched diol with 2 to 4 carbon atoms in the hydrocarbon chain;
b) 0.1-1.9 w/w of at least one further branched or unbranched aliphatic diol with 4 to 12 carbon atoms in the hydrocarbon chain that is different from a), whereby the hydrocarbon chain may be interrupted by an oxygen atom;
c) 0.5-10% w/w of at least one single-value aliphatic alcohol with 12 to 30 carbon atoms in the hydrocarbon chain.

2. Skin protection agent according to claim 1, **characterised in that** the same comprises at least one further compound d) in addition to a), b) and c), which carries at least one OH function.

3. Skin protection agent according to one of the claims 1 or 2, **characterised in that** the diols of component a) are selected from at least one aliphatic, unbranched diol with 2 to 4 carbon atoms in the hydrocarbon chain, and with two functional OH groups that comprise no further functional groups.

4. Skin protection agent according to one of the claims 1 to 3, **characterised in that** the diols of component b) are selected from: 1,2-alkan diols with 6 to 12 carbon atoms; and/or 1,3-alkan diols with 4 to 12 carbon atoms; and/or α-ω-alkan diols with 6 to 12 carbon atoms; and/or monoalkyl glycerines with 4 to 12 carbon atoms.

5. Skin protection agent according to one of the claims 1 to 4, **characterised in that** the alcohols of component c) are selected from linear, saturated, preferably primary alkanols with 12 to 30 carbon atoms in the hydrocarbon chain.

6. Skin protection agent according to one of the claims 1 to 5, **characterised in that** it further comprises component d), and **in that** compounds of component d) are selected from compounds that comprise at least one unsaturated hydrocarbon ring with 6, 7 or 8 carbon atoms.

7. Skin protection agent according to one of the claims 1 to 6, **characterised in that** it comprises a combination of components a) 1,2-propandiol, b) a mixture of 1,2-hexandiol and 1,2-octandiol, c) ceteary alcohol and optionally a compound of the component d), preferably at least bisabolol.

8. Skin protection agent according to one of the claims 1 to 7, **characterised in that** it further comprises an emulsifier mixture e) consisting of a combination of polyolpoly-12-hydroxy stearates with an alkyl and/or alkylene glucoside and a fat alcohol and/or partial glycerides and possible further co-emulsifiers, particularly preferably at least one ethoxylated dipolyhydroxy stearate.

9. Skin protection agent according to one of the claims 1 to 8, **characterised in that** it comprises:
a) 0.2 to 10% w/w of at least one aliphatic, unbranched diol with 2 to 4 carbon atoms in the hydrocarbon chain;
b) 0.1 to 1.9% w/w of at least one further aliphatic diol with 4 to 12 carbon atoms in the hydrocarbon chain that is different from a);
c) 0.5 to 10% w/w of at least one single-value aliphatic alcohol with 12 to 30 carbon atoms in the hydrocarbon chain;
d) 0.1 to 5% w/w of at least one further compound with 3 to 30 carbon atoms that carries at least one OH function;
e) 1 to 25% w/w of an emulsifier mixture selected from: PEG-30 glyceryl cocoate, PEG-6 caprylic/capric glyceride, sorbitan sesquicaprylate, sucrose cocoate, isoamyl cocoate polyglyceryl-3 caprate, PPG-3 myristyl ether, PPG-11 stearyl ether, cetearyl isononanoate, cetyl ethyl hexanoates, caprylic/capric triglyceride, decal cocoates, diethylhexyl carbonate, decyl oleate, PPG-15 stearyl ether, octyl dodecanol, isocetyl palmitate, isohexadecane, cetearyl ethyl hexanoate, isopropyl myristate, oleyl erucate, ethyl hexyl palmitate, ethyl hexyl stearate, isopropyl palmitate, PPG-14 butyl ether, triisostearine, C12-15 alkyl benzoate, cetyl ricenoleate, glyceryl ricenoleate, glyceryl stearate, isocetyl palmitate, isocetyl stearate, tocopheryl linoleate, methylheptyl isostearate and mixtures of the same;
f) 30 to 90% w/w water; and
g) further components of a quantity of 100% w/w - (sum of quantities of the components a) to f));
whereby the % w/w each relate to the total weight of the skin protection agent and add up to the sum of all component weights (% w/w), i.e. 100% w/w.

10. Skin protection agent according to one of the claims 1 to 9, **characterised in that** it comprises:
a) 0.2 to 10% w/w of at least one diol selected from: 1,2-propandiol; 1,3-propandiol; 1 ,2-ethandiol; 1,2-butandiol; 1,3-butandiol and/or 1,4-butandiol;
b) 0.1 to 1.9% w/w of at least one further aliphatic diol selected from: 1,2-hexandiol; 1,3-hexandiol; 1,6-hexandiol; 1,2-octandiol; 1,3-octandiol; 1,8-octandiol; 1,2-decandiol; 1,10-decandiol; 1,2-dodecandiol; glyceryl caprylate; methyl propane diol; ethyl hexyl glycerine; hexylene glycol that is different from a);
c) 0.5 to 10% w/w of at least one alcohol selected from: lauryl alcohol; cetyl alcohol; stearyl alcohol; ceryl alcohol and/or myricyl alcohol;
d) 0.1 to 5% w/w of at least one further compound selected from: bisabolol; 1,2-tropolone; capryl hydroxamic acid; glycerine; phenoxy ethanol; alpha-tocopherol; phenethyl alcohol and/or trideceth-8;
e) 1 to 25% w/w of an emulsifier mixture consisting of a combination of: polyolpoly-12-hydroxy stearates with an alkyl and/or alkylene glucoside and a fat alcohol and/or partial glyceride and possible further co-emulsifiers; particularly preferably at least one ethoxylated dipolyhydroxy stearate;
f) 50 to 85% w/w water;
g) further components of a quantity of 100% w/w - (sum of quantities of the components a) to f));
whereby the % w/w each relate to the total weight of the skin protection agent and add up to the sum of all component weights (% w/w), i.e. 100% w/w.

11. Skin protection agent according to one of the claims 1 to 10, **characterised in that** it is free from preservatives, whereby components a), b) and c) are not included under the term preservatives.

12. Methods for the production of stable multiple emulsions according to one of the claims 1 to 11, comprising the components of:
a) 0.2-10% w/w of at least one aliphatic unbranched diol with 2 to 4 carbon atoms in the hydrocarbon chain;
b) 0.1-1.9 w/w of_at least one further aliphatic diol with 4 to 12 carbon atoms in the hydrocarbon chain that is different from a), whereby the hydrocarbon chain may be interrupted by an oxygen atom; and
c) 0.5-10% w/w of at least one single-value aliphatic alcohol with 12 to 30 carbon atoms in the hydrocarbon chain; and
d) optionally at least one further compound with 3 to 30 carbon atoms that carries at least one OH function.

13. Use of a combination of:
a) 0.2-10% w/w of at least one aliphatic unbranched diol with 2 to 4 carbon atoms in the hydrocarbon chain;
b) 0.1-1.9 w/w of_at least one further branched or unbranched aliphatic diol with 4 to 12 carbon atoms in the hydrocarbon chain that is different from a), whereby the hydrocarbon chain may be interrupted by an oxygen atom;
c) 0.5-10% w/w of_at least one single-value aliphatic alcohol with 12 to 30 carbon atoms in the hydrocarbon chain; and
d) optionally at least one further component that carries at least one OH function, in a skin protection agent for the stabilisation of W/O/W emulsions.

14. Non-therapeutic use of a combination of:
a) 0.2-10% w/w of_at least one aliphatic unbranched diol with 2 to 4 carbon atoms in the hydrocarbon chain;
b) 0.1-1.9 w/w of_at least one further branched or unbranched aliphatic diol with 4 to 12 carbon atoms in the hydrocarbon chain that is different from a), whereby the hydrocarbon chain may be interrupted by an oxygen atom;
c) 0.5-10% w/w of at least one single-value aliphatic alcohol with 12 to 30 carbon atoms in the hydrocarbon chain; and
d) optionally at least one further component that carries at least one OH function, in skin protection agents in the form of W/O/W multiple emulsions for improved protection against aqueous noxa.

15. Use of a skin protection agent according to one of the claims 1 to 11 for non-therapeutic topical application on the skin.

## Patentansprüche

1. Hautschutzmittel in Form einer Wasser-in-ÖI-in-Wasser (W/O/W)-Emulsion, umfassend:
a) 0,2-10% Gew.-% mindestens eines unverzweigten aliphatischen Diols mit 2 bis 4 Kohlenstoffatomen in der Kohlenwasserstoffkette;
b) 0,1-1,9 Gew.-% mindestens eines weiteren verzweigten oder unverzweigten aliphatischen Diols mit 4 bis 12 Kohlenstoffatomen in der Kohlenwasserstoffkette, das von a) verschieden ist, wobei die Kohlenwasserstoffkette durch ein Sauerstoffatom unterbrochen sein kann;
c) 0,5-10 % Gew.-% mindestens eines einwertigen aliphatischen Alkohols mit 12 bis 30 Kohlenstoffatomen in der Kohlenwasserstoffkette.

2. Hautschutzmittel nach Anspruch 1, **dadurch gekennzeichnet, dass** es zusätzlich zu a), b) und c) mindestens eine weitere Verbindung d) enthält, die mindestens eine OH-Funktion trägt.

3. Hautschutzmittel nach einem der Ansprüche 1 oder 2, **dadurch gekennzeichnet, dass** die Diole der Komponente a) ausgewählt sind aus mindestens einem unverzweigten aliphatischen Diol mit 2 bis 4 Kohlenstoffatomen in der Kohlenwasserstoffkette, und mit zwei funktionellen OH-Gruppen, die keine weiteren funktionellen Gruppen umfassen.

4. Hautschutzmittel nach einem der Ansprüche 1 bis 3, **dadurch gekennzeichnet, dass** die Diole der Komponente b) ausgewählt sind aus: 1,2-Alkandiolen mit 6 bis 12 Kohlenstoffatomen; und/oder 1,3-Alkandiolen mit 4 bis 12 Kohlenstoffatomen; und/oder α-ω-Alkandiolen mit 6 bis 12 Kohlenstoffatomen; und/oder Monoalkylglycerinen mit 4 bis 12 Kohlenstoffatomen.

5. Hautschutzmittel nach einem der Ansprüche 1 bis 4, **dadurch gekennzeichnet, dass** die Alkohole der Komponente c) ausgewählt sind aus linearen, gesättigten, vorzugsweise primären Alkanolen mit 12 bis 30 Kohlenstoffatomen in der Kohlenwasserstoffkette.

6. Hautschutzmittel nach einem der Ansprüche 1 bis 5, **dadurch gekennzeichnet, dass** es des Weiteren die Komponente d) umfasst, und dass die Verbindungen der Komponente d) ausgewählt sind aus Verbindungen, die mindestens einen ungesättigten Kohlenwasserstoffring mit 6, 7 oder 8 Kohlenstoffatomen aufweisen.

7. Hautschutzmittel nach einem der Ansprüche 1 bis 6, **dadurch gekennzeichnet, dass** es eine Kombination der Komponenten a) 1,2-Propandiol, b) ein Gemisch aus 1,2-Hexandiol und 1,2-Octandiol, c) Cetearylalkohol und gegebenenfalls eine Verbindung der Komponente d), vorzugsweise mindestens Bisabolol, umfasst.

8. Hautschutzmittel nach einem der Ansprüche 1 bis 7, **dadurch gekennzeichnet, dass** es des Weiteren eine Emulgatormischung e), bestehend aus einer Kombination von Polyolpoly-12-Hydroxystearaten mit einem Alkyl- und/oder Alkylenglucosid und einem Fettalkohol und/oder Partialglyceriden und möglichen weiteren Co-Emulgatoren, besonders bevorzugt mindestens einem ethoxylierten Dipolyhydroxystearat, umfasst.

9. Hautschutzmittel nach einem der Ansprüche 1 bis 8, **dadurch gekennzeichnet, dass** es umfasst:
a) 0,2 bis 10 Gew.-% mindestens eines unverzweigten aliphatischen Diols mit 2 bis 4 Kohlenstoffatomen in der Kohlenwasserstoffkette;
b) 0,1 bis 1,9 Gew.-% mindestens eines weiteren aliphatischen Diols mit 4 bis 12 Kohlenstoffatomen in der Kohlenwasserstoffkette, das von a) verschieden ist;
c) 0,5 bis 10 Gew.-% mindestens eines einwertigen aliphatischen Alkohols mit 12 bis 30 Kohlenstoffatomen in der Kohlenwasserstoffkette;
d) 0,1 bis 5 Gew.-% mindestens einer weiteren Verbindung mit 3 bis 30 Kohlenstoffatomen, die mindestens eine OH-Funktion trägt;
e) 1 bis 25 Gew.-% einer Emulgatormischung, ausgewählt aus: PEG-30-Glycerylcocoat, PEG-6-Capryl-/Caprin-Glycerid, Sorbitansesquicaprylat, Saccharosecocoat, Isoamylcocoat, Polyglyceryl-3-Caprat, PPG-3-Myristylether, PPG-11-Stearylether, Cetearylisononanoat, Cetylethylhexanoate, Capryl-/Caprin-Triglycerid, Decylcocoate, Diethylhexylcarbonat, Decyloleat, PPG-15- Stearylether, Octyldodecanol, Isocetylpalmitat, Isohexadecan, Cetearylethylhexanoat, Isopro-pylmyristat, Oleylerucat, Ethylhexylpalmitat, Ethylhexylstearat, Isopropylpalmitat, PPG-14-Butylether, Triisostearin, C12-15-Alkylbenzoat, Cetylricenoleat, Glycerylricenoleat, Glycerylstearat, Isocetylpalmitat, Isocetylstearat, Tocopheryl-Linoleat, Methylheptylisostearat und Mischungen davon;
f) 30 bis 90 Gew.-% Wasser; und
g) weitere Komponenten in einer Menge von 100 Gew.-% (Summe der Mengen der Komponenten a) bis f));
wobei sich die Gewichtsprozentangaben jeweils auf das Gesamtgewicht des Hautschutzmittels beziehen und insgesamt die Summe aller Komponentengewichte (Gew.-%) ergeben, d.h. 100 Gew.-%.

10. Hautschutzmittel nach einem der Ansprüche 1 bis 9, **dadurch gekennzeichnet, dass** es umfasst:
a) 0,2 bis 10 Gew.-% mindestens eines Diols, ausgewählt aus: 1,2-Propandiol; 1,3-Propandiol; 1,2-Ethandiol; 1,2-Butandiol; 1,3-Butandiol und/oder 1,4-Butandiol;
b) 0,1 bis 1,9 Gew.-% mindestens eines weiteren aliphatischen Diols, ausgewählt aus: 1,2-Hexandiol; 1,3-Hexandiol; 1,6-Hexandiol; 1,2-Octandiol; 1,3-Octandiol; 1,8-Octandiol; 1,2-Decandiol; 1,10-Decandiol; 1,2-Dodecandiol; Glycerylcaprylat; Methylpropandiol; Ethylhexylglycerin; Hexylenglycol, das von a) verschieden ist;
c) 0,5 bis 10 Gew.-% mindestens eines Alkohols, ausgewählt aus: Laurylalkohol; Cetylalkohol; Stearylalkohol; Cerylalkohol und/oder Myricylalkohol;
d) 0,1 bis 5 Gew.-% mindestens einer weiteren Verbindung, ausgewählt aus: Bisabolol; 1,2-Tropolon; Caprylhydroxamsäure; Glycerin; Phenoxyethanol; alpha-Tocopherol; Phenethylalkohol und/oder Trideceth-8;
e) 1 bis 25 Gew.-% einer Emulgatormischung, bestehend aus einer Kombination von: Polyolpoly-12-Hydroxystearaten mit einem Alkyl- und/oder Alkylenglucosid und einem Fettalkohol und/oder einem Partialglycerid und möglichen weiteren Co-Emulgatoren; besonders bevorzugt mindestens einem ethoxylierten Dipolyhydroxystearat;
f) 50 bis 85 % Gew.-% Wasser;
g) weitere Komponenten in einer Menge von 100 Gew.-% (Summe der Mengen der Komponenten a) bis f));
wobei sich die Gewichtsprozentangaben jeweils auf das Gesamtgewicht des Hautschutzmittels beziehen und insgesamt die Summe aller Komponentengewichte (Gew.-%) ergeben, d.h. 100 Gew.-%.

11. Hautschutzmittel nach einem der Ansprüche 1 bis 10, **dadurch gekennzeichnet, dass** es frei von Konservierungsmitteln ist, wobei die Komponenten a), b) und c) nicht unter den Begriff Konservierungsmittel fallen.

12. Verfahren zur Herstellung von stabilen Mehrfachemulsionen nach einem der Ansprüche 1 bis 11, umfassend die Komponenten:
a) 0,2-10 % Gew.-% mindestens eines unverzweigten aliphatischen Diols mit 2 bis 4 Kohlenstoffatomen in der Kohlenwasserstoffkette;
b) 0,1-1,9 Gew.-% mindestens eines weiteren aliphatischen Diols mit 4 bis 12 Kohlenstoffatomen in der Kohlenwasserstoffkette, das von a) verschieden ist, wobei die Kohlenwasserstoffkette durch ein Sauerstoffatom unterbrochen sein kann; und
c) 0,5-10 % Gew.-% mindestens eines einwertigen aliphatischen Alkohols mit 12 bis 30 Kohlenstoffatomen in der Kohlenwasserstoffkette; und
d) gegebenenfalls mindestens eine weitere Verbindung mit 3 bis 30 Kohlenstoffatomen, die mindestens eine OH-Funktion trägt.

13. Verwendung einer Kombination aus:
a) 0,2-10 % Gew.-% mindestens eines unverzweigten aliphatischen Diols mit 2 bis 4 Kohlenstoffatomen in der Kohlenwasserstoffkette;
b) 0,1-1,9 Gew.-% mindestens eines weiteren verzweigten oder unverzweigten aliphatischen Diols mit 4 bis 12 Kohlenstoffatomen in der Kohlenwasserstoffkette, das von a) verschieden ist, wobei die Kohlenwasserstoffkette durch ein Sauerstoffatom unterbrochen sein kann;
c) 0,5-10 Gew.-% mindestens eines einwertigen aliphatischen Alkohols mit 12 bis 30 Kohlenstoffatomen in der Kohlenwasserstoffkette; und
d) gegebenenfalls mindestens einer weiteren Komponente, die mindestens eine OH-Funktion trägt,
in einem Hautschutzmittel zur Stabilisierung von W/O/W-Emulsionen.

14. Nicht-therapeutische Verwendung einer Kombination aus:
a) 0,2-10 Gew.-% mindestens eines unverzweigten aliphatischen Diols mit 2 bis 4 Kohlenstoffatomen in der Kohlenwasserstoffkette;
b) 0,1-1,9 Gew.-% mindestens eines weiteren verzweigten oder unverzweigten aliphatischen Diols mit 4 bis 12 Kohlenstoffatomen in der Kohlenwasserstoffkette, das von a) verschieden ist, wobei die Kohlenwasserstoffkette durch ein Sauerstoffatom unterbrochen sein kann;
c) 0,5-10 Gew.-% mindestens eines einwertigen aliphatischen Alkohols mit 12 bis 30 Kohlenstoffatomen in der Kohlenwasserstoffkette; und
d) gegebenenfalls mindestens einer weiteren Komponente, die mindestens eine OH-Funktion trägt,
in Hautschutzmitteln in Form von W/O/W-Mehrfachemulsionen zum verbesserten Schutz gegen wässrige Noxen.

15. Verwendung eines Hautschutzmittels nach einem der Ansprüche 1 bis 11 zur nichttherapeutischen topischen Anwendung auf der Haut.

## Revendications

1. Agent de protection de la peau, sous la forme d'une émulsion eau dans huile dans eau (W/O/W), comprenant :
a) 0,2 à 10 % p/p d'au moins un diol aliphatique non ramifié contenant 2 à 4 atomes de carbone dans la chaîne hydrocarbonée ;
b) 0,1 à 1,9 p/p d'au moins un diol aliphatique ramifié ou non ramifié supplémentaire contenant 4 à 12 atomes de carbone dans la chaîne hydrocarbonée qui est différent de a), selon lequel la chaîne hydrocarbonée peut être interrompue par un atome d'oxygène ;
c) 0,5 à 10 % p/p d'au moins un alcool aliphatique monovalent contenant 12 à 30 atomes de carbone dans la chaîne hydrocarbonée.

2. Agent de protection de la peau selon la revendication 1, **caractérisé en ce que** celui-ci comprend au moins un composé d) supplémentaire en plus de a), b) et c), qui porte au moins une fonction OH.

3. Agent de protection de la peau selon l'une des revendications 1 ou 2, **caractérisé en ce que** les diols du composant a) sont sélectionnés parmi au moins un diol aliphatique non ramifié contenant 2 à 4 atomes de carbone dans la chaîne hydrocarbonée, et contenant deux groupes OH fonctionnels qui ne comprennent pas de groupes fonctionnels supplémentaires.

4. Agent de protection de la peau selon l'une des revendications 1 à 3, **caractérisé en ce que** les diols du composant b) sont sélectionnés parmi : les 1,2-alcanediols contenant 6 à 12 atomes de carbone ; et/ou les 1,3-alcanediols contenant 4 à 12 atomes de carbone ; et/ou les α-ω-alcanediols contenant 6 à 12 atomes de carbone ; et/ou les monoalkylglycérines contenant 4 à 12 atomes de carbone.

5. Agent de protection de la peau selon l'une des revendications 1 à 4, **caractérisé en ce que** les alcools du composant c) sont sélectionnés parmi les alcanols linéaires, saturés, de préférence primaires, contenant 12 à 30 atomes de carbone dans la chaîne hydrocarbonée.

6. Agent de protection de la peau selon l'une des revendications 1 à 5, **caractérisé en ce qu'**il comprend en outre le composant d), et **en ce que** les composés du composant d) sont sélectionnés parmi les composés qui comprennent au moins un cycle hydrocarboné insaturé contenant 6, 7 ou 8 atomes de carbone.

7. Agent de protection de la peau selon l'une des revendications 1 à 6, **caractérisé en ce qu'**il comprend une combinaison de composants a) le 1,2-propanediol, b) un mélange de 1,2-hexanediol et 1,2-octanediol, c) l'alcool cétéarylique et facultativement un composé du composant d), de préférence au moins le bisabolol.

8. Agent de protection de la peau selon l'une des revendications 1 à 7, **caractérisé en ce qu'**il comprend en outre un mélange émulsifiant e) consistant en une combinaison de poly-12-hydroxystéarates de polyol avec un glucoside d'alkyle et/ou d'alkylène et un alcool gras et/ou des glycérides partiels et des co-émulsifiants supplémentaires possibles, de manière particulièrement préférée au moins un dipolyhydroxystéarate éthoxylé.

9. Agent de protection de la peau selon l'une des revendications 1 à 8, **caractérisé en ce qu'**il comprend :
a) 0,2 à 10 % p/p d'au moins un diol aliphatique non ramifié contenant 2 à 4 atomes de carbone dans la chaîne hydrocarbonée ;
b) 0,1 à 1,9 % p/p d'au moins un diol aliphatique supplémentaire contenant 4 à 12 atomes de carbone dans la chaîne hydrocarbonée qui est différent de a) ;
c) 0,5 à 10 % p/p d'au moins un alcool aliphatique monovalent contenant 12 à 30 atomes de carbone dans la chaîne hydrocarbonée ;
d) 0,1 à 5 % p/p d'au moins un composé supplémentaire contenant 3 à 30 atomes de carbone qui porte au moins une fonction OH ;
e) 1 à 25 % p/p d'un mélange émulsifiant sélectionné parmi : le cocoate de glycéryle PEG-30, le glycéride caprylique/caprique PEG-6, le sesquicaprylate de sorbitane, le cocoate de sucrose, le cocoate d'isoamyle, le caprate de polyglycéryle-3, l'éther myristylique de PPG-3, l'éther stéarylique de PPG-11, l'isononanoate de cétéaryle, les hexanoates de cétyléthyle, le triglycéride caprylique/caprique, les cocoates de décal, le carbonate de diéthylhexyle, l'oléate de décyle, l'éther stéarylique de PPG-15, l'octyldodécanol, le palmitate d'isocétyle, l'isohexadécane, l'hexanoate de cétéaryléthyle, le myristate d'isopropyle, l'érucate d'oléyle, le palmitate d'éthylhexyle, le stéarate d'éthylhexyle, le palmitate d'isopropyle, l'éther butylique de PPG-14, la triisostéarine, le benzoate d'alkyle en C12-15, le ricinoléate de cétyle, le ricinoléate de glycéryle, le stéarate de glycéryle, le palmitate d'isocétyle, le stéarate d'isocétyle, le linoléate de tocophéryle, l'isostéarate de méthylheptyle et les mélanges de ceux-ci ;
f) 30 à 90 % p/p d'eau ; et
g) des composants supplémentaires d'une quantité de 100 % p/p (somme de quantités des composants a) à f)) ;
selon lequel les % p/p se rapportent chacun au poids total de l'agent de protection de la peau et s'additionnent en la somme de tous les poids de composants (% p/p), c.-à-d. 100 % p/p.

10. Agent de protection de la peau selon l'une des revendications 1 à 9, **caractérisé en ce qu'**il comprend :
a) 0,2 à 10 % p/p d'au moins un diol sélectionné parmi : le 1,2-propandiol ; le 1,3-propandiol ; le 1,2-éthanediol ; le 1,2-butanediol ; le 1,3-butanediol et/ou le 1,4-butanediol ;
b) 0,1 à 1,9 % p/p d'au moins un diol aliphatique supplémentaire sélectionné parmi : le 1,2-hexanediol ; le 1,3-hexanediol ; le 1,6-hexanediol ; le 1,2-octanediol ; le 1,3-octanediol ; le 1,8-octanediol ; le 1,2-décanediol ; le 1,10-décanediol ; le 1,2-dodécanediol ; le caprylate de glycéryle ; le méthylpropanediol ; l'éthylhexylglycérine ; l'hexylène glycol qui est différent de a) ;
c) 0,5 à 10 % p/p d'au moins un alcool sélectionné parmi ; l'alcool laurylique ; l'alcool cétylique ; l'alcool stéarylique ; l'alcool cérylique et/ou l'alcool myricylique ;
d) 0,1 à 5 % p/p d'au moins un composé supplémentaire sélectionné parmi : le bisabolol ; la 1,2-tropolone ; l'acide caprylhydroxamique ; la glycérine ; le phénoxyéthanol ; l'alpha-tocophérol ; l'alcool phénéthylique et/ou le tridécéth-8 ;
e) 1 à 25 % p/p d'un mélange émulsifiant consistant en une combinaison de : poly-12-hydroxystéarates de polyol avec un glucoside d'alkyle et/ou d'alkylène et un alcool gras et/ou un glycéride partiel et des co-émulsifiants supplémentaires possibles ; de manière particulièrement préférée au moins un dipolyhydroxystéarate éthoxylé ;
f) 50 à 85 % p/p d'eau;
g) des composants supplémentaires d'une quantité de 100 % p/p (somme de quantités des composants a) à f)) ;
selon lequel les % p/p se rapportent chacun au poids total de l'agent de protection de la peau et s'additionnent en la somme de tous les poids de composants (% p/p), c.-à-d. 100 % p/p.

11. Agent de protection de la peau selon l'une des revendications 1 à 10, **caractérisé en ce qu'**il est exempt de conservateurs, les composants a), b) et c) n'étant pas inclus dans le terme conservateurs.

12. Procédés pour la production d'émulsions multiples stables selon l'une des revendications 1 à 11, comprenant les composants de :
a) 0,2 à 10 % p/p d'au moins un diol aliphatique non ramifié contenant 2 à 4 atomes de carbone dans la chaîne hydrocarbonée ;
b) 0,1 à 1,9 p/p d'au moins un diol aliphatique supplémentaire contenant 4 à 12 atomes de carbone dans la chaîne hydrocarbonée qui est différent de a), selon lequel la chaîne hydrocarbonée peut être interrompue par un atome d'oxygène ; et
c) 0,5 à 10 % p/p d'au moins un alcool aliphatique monovalent contenant 12 à 30 atomes de carbone dans la chaîne hydrocarbonée ; et
d) facultativement au moins un composé supplémentaire contenant 3 à 30 atomes de carbone qui porte au moins une fonction OH.

13. Utilisation d'une combinaison de :
a) 0,2 à 10 % p/p d'au moins un diol aliphatique non ramifié contenant 2 à 4 atomes de carbone dans la chaîne hydrocarbonée ;
b) 0,1 à 1,9 p/p d'au moins un diol aliphatique ramifié ou non ramifié supplémentaire contenant 4 à 12 atomes de carbone dans la chaîne hydrocarbonée qui est différent de a), selon laquelle la chaîne hydrocarbonée peut être interrompue par un atome d'oxygène ;
c) 0,5 à 10 % p/p d'au moins un alcool aliphatique monovalent contenant 12 à 30 atomes de carbone dans la chaîne hydrocarbonée ; et
d) facultativement au moins un composant supplémentaire qui porte au moins une fonction OH,
dans un agent de protection de la peau pour la stabilisation d'émulsions W/O/W.

14. Utilisation non thérapeutique d'une combinaison de :
a) 0,2 à 10 % p/p d'au moins un diol aliphatique non ramifié contenant 2 à 4 atomes de carbone dans la chaîne hydrocarbonée ;
b) 0,1 à 1,9 p/p d'au moins un diol aliphatique ramifié ou non ramifié supplémentaire contenant 4 à 12 atomes de carbone dans la chaîne hydrocarbonée qui est différent de a), selon laquelle la chaîne hydrocarbonée peut être interrompue par un atome d'oxygène ;
c) 0,5 à 10 % p/p d'au moins un alcool aliphatique monovalent contenant 12 à 30 atomes de carbone dans la chaîne hydrocarbonée ; et
d) facultativement au moins un composant supplémentaire qui porte au moins une fonction OH,
dans des agents de protection de la peau sous la forme d'émulsions multiples W/O/W pour une protection améliorée contre des noxa aqueux.

15. Utilisation d'un agent de protection de la peau selon l'une des revendications 1 à 11 pour une application topique non thérapeutique sur la peau.
